# FASCICULE DE BREVET EUROPEEN

(11) **EP 3 344 596 B1**
(45) Date de publication et mention de la délivrance du brevet: **20.11.2019**
(21) Numéro de dépôt: 16758169.3
(22) Date de dépôt: 30.08.2016
(51) Int. Cl.: C07C 1/24, C07C 5/333, C07C 11/08, C07C 11/167

(54) **PROCEDE INTEGRE DE PRODUCTION DE BUTADIENE A PARTIR DE BUTANOL**
VERFAHREN ZUR HERSTELLUNG VON BUTADIEN AUS BUTANOL
INTEGRATED METHOD FOR PRODUCING BUTADIENE FROM BUTANOL

(30) Priorité: 03.09.2015 FR 1558190
(43) Date de publication de la demande: 11.07.2018
(73) Titulaire: IFP Energies nouvelles, 92500 Rueil-Malmaison (FR); Compagnie Générale des Etablissements Michelin, 63000 Clermont-Ferrand (FR)
(72) Inventeur: THINON, Olivier, 42300 Roanne (FR); COUPARD, Vincent, 69100 Villeurbanne (FR); HUYGHE, Raphael, 69700 Saint Andeol Le Chateau (FR)
(74) Mandataire: IFP Energies nouvelles
(86) Numéro de dépôt international: PCT/EP2016/070424
(87) Numéro de publication internationale: WO 2017/037067

(56) Documents cités:
- US-A1- 2010 216 958
- US-B2- 8 450 543

## Description

### DOMAINE DE L'INVENTION

L'invention est relative à la production de 1,3-butadiène à partir de n-butanol. Ledit n-butanol est déshydraté pour conduire au butène qui est lui-même déshydrogéné pour conduire au 1,3-butadiène désiré.

### ART ANTÉRIEUR

La production de 1,3-butadiène est généralement opérée par l'intermédiaire du procédé de vapocraquage. Cependant le procédé de vapocraquage est non sélectif et les rendements en 1,3-butadiène dépendent de la charge traitée. La déshydrogénation des butènes permet de s'affranchir de ces limitations lors de la production de 1,3-butadiène.

La réaction de déshydrogénation des butènes peut être opérée selon deux voies différentes:
- une voie réductrice, dans laquelle les butènes sont déshydrogénés au contact d'un catalyseur pour produire du 1,3-butadiène et de l'hydrogène. La déshydrogénation par voie réductrice est endothermique et équilibrée, elle est réalisée à faible pression partielle de butènes (sous vide partiel ou avec une forte dilution à la vapeur) et à haute température, généralement entre 550 et 700°C ;
- une voie oxydante, dans laquelle les butènes sont mis en contact avec un catalyseur et un agent oxydant tel que l'air, l'oxygène, un oxyde solide ou encore le CO₂ pour donner du 1,3-butadiène et de l'eau. La déshydrogénation par voie oxydante est très exothermique et non équilibrée, elle est généralement réalisée aux alentours de la pression atmosphérique, avec une forte dilution à la vapeur entre 300 et 600°C.

Les procédés de déshydrogénation des butènes en 1,3-butadiène nécessitent un apport conséquent en vapeur de dilution. Par ailleurs, la présence d'iso-butène dans la charge est préjudiciable aux performances catalytiques en raison d'une formation plus importante de coke sur le catalyseur ainsi qu'une formation de composés légers tels que le méthane ou le CO₂. Les iso-butènes nuisent également à la rentabilité économique du procédé par la surconsommation d'oxygène et la formation de composés oxygénés lourds non désirés qu'il est nécessaire de séparer. Les n-butènes sont ainsi des charges privilégiées pour la production de 1,3-butadiène par déshydrogénation.

Le brevet US 8 450 543 décrit la production de composés chimiques bio-sourcés, dont le 1,3-butadiène, à partir d'iso-butanol selon un schéma intégré de plusieurs procédés. La production de 1,3-butadiène est réalisée à partir d'une première étape de déshydratation de l'iso-butanol en un mélange de n-butènes et d'iso-butène. La charge iso-butanol est obtenue par fermentation puis séparation, et contient plus de 98,8% en poids d'iso-butanol, de l'eau et du 3-méthylbutan-1-ol. Les butènes sont ensuite séparés de l'eau et de l'iso-butanol non converti, et traités dans une seconde étape de déshydrogénation pour produire du 1,3-butadiène. Le brevet US 8 450 543 indique que l'iso-butène est inerte dans les conditions de déshydrogénation des butènes linéaires. Aussi, il est mentionné un mode de réalisation où l'effluent réactionnel de la déshydratation peut être envoyé sans séparation intermédiaire dans le réacteur de déshydrogénation, dans lequel l'iso-butène ne réagit pas.

Le brevet US 3 895 049 décrit un procédé de production simultanée de 1,3-butadiène et de méthacroléoine à partir d'un mélange de n-butènes et d'iso-butène avec un catalyseur de type BiMo connu pour son activité pour la déshydrogénation oxydante des n-butènes en 1,3-butadiène et dans les conditions classiquement reportées pour la déshydrogénation oxydante comme une température comprise entre 300°C et 550°C et un ratio molaire oxygène sur butènes entre 0,5 et 5.

Le brevet US 2 956 092 divulgue que la production de 1,3-butadiène diminue à partir de 3% d'iso-butane et d'iso-butène dans la charge de déshydrogénation. Les brevets US 2 956 092 et US 4 504 692 divulguent ainsi des procédés de production de butadiène à partir de charge butane et butènes avec une étape de séparation des composés iso-butane et iso-butène.

La demande de brevet US 2014/296588 décrit un procédé de production de butadiène à partir de composés oxygénés en une succession d'étapes de conversion et de séparation. La charge oxygénée du procédé est un alcool contenant de 1 à 5 atomes de carbone et de préférence du méthanol. La première étape est une étape de conversion catalytique de la charge alcool en oléfine, tel que le procédé communément appelé Methanol To Olefin (MTO) dans la dénomination anglaise. Cette étape produit un effluent contenant des oléfines ayant entre 2 et 5 atomes de carbone. Une première étape de séparation permet de récupérer les oléfines ayant 4 atomes de carbone incluant les n-butènes et l'iso-butène. L'iso-butène est récupéré par éthérification dans une seconde étape de séparation afin d'éviter la formation de composés non désirés en déshydrogénation. La charge butènes exempt d'iso-butène ainsi obtenue est ensuite envoyée dans une étape de déshydrogénation oxydante pour la production de 1,3-butadiène. Ce procédé inclut de nombreuses étapes avec une étape de production de butènes non sélective.

US 2010/216958 décrit un procédé de production de butadiène comprenant une réaction de déshydratation de butanol et une étape de déshydrogénation en utilisant un catalyseur comprenant une alumine.

### OBJET DE L'INVENTION

L'invention concerne un procédé intégré thermiquement de production de butadiène à partir de butanol comprenant au moins les étapes suivantes:
a) déshydratation du butanol alimentée par une alimentation de déshydratation formée à partir d'au moins ladite charge n-butanol diluée avec au moins une partie de l'effluent eau purifiée issu de l'étape c), conduisant à un effluent butènes dans au moins un réacteur en présence d'un catalyseur comprenant une alumine,
b) déshydrogénation oxydante alimentée par au moins ledit effluent butènes, dilué avec au moins une partie de l'effluent eau purifiée issu de l'étape c), en butadiène, ledit effluent butènes n'ayant subi aucun traitement à la suite de l'étape a) de déshydratation,
c) séparation de l'effluent issu de l'étape b) en au moins un effluent butadiène et un effluent eau purifiée,
dans lequel au moins une partie de l'énergie thermique nécessaire est apportée par la réaction de déshydrogénation de l'étape b) la température élevée de l'effluent butadiène en sortie de l'étape de déshydrogénation permettant de préchauffer ou chauffer les flux entrant et/ou sortant de l'étape a) de déshydratation et de vaporiser la charge n-butanol et de générer une partie de la vapeur de dilution.

Plus particulièrement, l'invention concerne un procédé intégré thermiquement de production de butadiène à partir d'une charge comprenant au moins 60% poids de butanol choisi parmi le n-butan-1-ol et le n-butan-2-ol seuls ou en mélange, comprenant au moins :
a) une première étape réactionnelle de déshydratation de ladite charge préalablement dilué avec au moins une partie de l'effluent eau purifiée issu de l'étape c) en un effluent butènes dans au moins un réacteur, opérée à une température à l'entrée de ladite étape a) comprise entre 300 et 450°C, à une pression comprise entre 0,2 et 2 MPa, à une vitesse pondérale horaire comprise entre 0,5 et 14 h⁻¹, de manière préférée entre 0,5 et 5 h⁻¹ et de manière encore plus préférée entre 1 et 5 h⁻¹ ladite étape étant réalisée en présence d'un catalyseur comprenant une alumine ;
b) une seconde étape réactionnelle de déshydrogénation oxydante dudit effluent butènes produit lors de ladite première étape, dilué avec au moins une partie de l'effluent eau purifiée issu de l'étape c), en un effluent butadiène, ledit effluent butènes n'ayant subi aucun traitement à la suite de l'étape a) de déshydratation, ladite étape réactionnelle de déshydrogénation oxydante étant opérée avec un ratio molaire n-butènes/dioxygène compris entre 0,5 et 3, de préférence entre 0,5 et 1, la température en entrée de ladite étape réactionnelle de déshydrogénation oxydante étant d'au moins 300°C.
c) une troisième étape de séparation en au moins un effluent contenant le butadiène et un effluent comprenant l'eau purifiée,
Le procédé selon l'invention est avantageux du fait des conversions et sélectivités en n-butènes du catalyseur de déshydratation particulièrement élevées. L'effluent butènes produit à l'étape de déshydratation contient une très faible quantité d'impuretés telles que l'iso-butène et des composés oxygénés, permettant d'opérer l'étape de déshydrogénation sans problèmes et sans nécessité de purification intermédiaire. De plus, il est ainsi possible de co-alimenter l'étape de déshydrogénation avec des charges butènes contenant plus d'impuretés, par exemple plus de 3 % en poids d'iso-butène par rapport aux butènes, qui ne pourraient pas être traités directement en déshydrogénation sans un prétraitement pour éliminer les impuretés, lesdites charges butènes pouvant être d'origine fossile ou encore issus de la biomasse.

Un autre avantage du procédé selon l'invention est la propriété dudit catalyseur de déshydratation de jouer le rôle de masse de captation des impuretés contenues dans la charge, en particulier des impuretés azotés et soufrés, pouvant nuire aux performances du catalyseur de déshydrogénation et / ou aux spécifications de pureté requises pour le 1,3-butadiène produit. Par exemple, il est indiqué dans le « Butadiene Product Stewardship Guidance Manual » une spécification en soufre et azote respectivement de 5 et 2 ppm dans le 1,3-butadiène produit.

Un avantage supplémentaire du procédé selon l'invention est la diminution de la consommation de vapeur d'eau d'origine externe au procédé pour assurer les performances de la déshydrogénation grâce à l'utilisation de la vapeur d'eau formée par la réaction de déshydratation. Ladite vapeur d'eau formée par la réaction de déshydratation permet de réaliser une dilution supplémentaire de l'effluent butènes avant son introduction dans le réacteur de déshydrogénation.

Un avantage certain du procédé selon l'invention est la faible consommation énergétique du fait d'une intégration thermique des étapes de déshydratation et de déshydrogénation. En particulier, la température élevée de l'effluent butadiène en sortie de l'étape c) de déshydrogénation permet de préchauffer ou chauffer les flux entrant et/ou sortant de l'étape de déshydratation et de vaporiser la charge n-butanol et de générer une partie de la vapeur de dilution.

Un autre avantage du procédé selon l'invention, par rapport à l'utilisation de deux procédés indépendants de déshydratation puis de déshydrogénation, est de permettre la mutualisation des équipements intervenant dans la boucle d'eau, à savoir la colonne de refroidissement dans laquelle se fait la séparation de l'effluent gazeux contenant les butènes et butadiène, et de l'effluent eau issus de l'étape c), et l'unité de purification de l'eau de recycle. Cette mutualisation se traduit par un gain important sur la consommation des utilités pour refroidir les boucles de recycle dans la colonne de refroidissement et sur l'énergie nécessaire à la purification de l'eau récupérée.

### DESCRIPTION DÉTAILLÉE DE L'INVENTION

### Charge

La charge n-butanol traitée dans le procédé selon l'invention comprend au moins 60% poids de n-butanol et de 0 à 40% poids d'eau. On entend par n-butanol le n-butan-1-ol et le n-butan-2-ol seuls ou en mélange.

Le n-butanol peut être produit par différents procédés tel que, par exemple, des procédés de fermentation, des procédés de gazéification suivi de la conversion du gaz de synthèse vers les alcools, des procédés d'hydroformylation du propylène (propylène + CO + H₂) en butyraldéhyde suivi d'une hydrogénation sélective en butanol, des procédés de conversion d'éthanol en butanol.

La charge n-butanol provient de préférence de ressources renouvelables. Par exemple, la charge n-butanol peut être produite par fermentation de sucres obtenus à partir de cultures de plantes sucrières comme la canne à sucre, les betteraves, ou encore les plantes amylacées ou du traitement de la biomasse lignocellulosique ou de cellulose hydrolysée. Les micro-organismes du type Clostridium *acetobutylicum* sont connus pour réaliser la fermentation des sucres en n-butanol.

La charge n-butanol peut également provenir de la recombinaison chimique ou biologique des gaz de synthèse produits par la gazéification de la biomasse ou de tous déchets organiques viables pour la gazéification tels que par exemple les résidus agricoles, résidus forestiers, déchets ménagers, pneumatiques usés, boues d'épuration.

La charge n-butanol peut contenir, en plus d'une quantité variables d'eau, des impuretés azotés et soufrés, tel que par exemple la 2-méthylpyridine, le 2-N-propylthiophène, le diméthyltrisulfure et le carbone disulfure. Des concentrations de ces composés entre 0,1 et 5 mg/L sont généralement mesurées dans le n-butanol produit par fermentation.

### Étape de déshydratation

Conformément à l'invention, ladite charge n-butanol est déshydratée en un effluent butènes dans au moins un réacteur.

La réaction de déshydratation correspond schématiquement à la réaction suivante : C₄H₁₀O → C₄H₈ + H₂O.

Ladite étape de déshydratation peut être réalisée au sein d'au moins un réacteur isotherme ou adiabatique comprenant un lit de catalyseur de déshydratation, ledit lit étant fixe ou mobile.

Ladite étape de déshydratation est avantageusement réalisée au sein de deux réacteurs successifs isothermes ou adiabatiques comprenant chacun un lit de catalyseur de déshydratation, ledit lit étant fixe ou mobile. L'utilisation de deux lits successifs permet d'améliorer l'opérabilité de ladite étape en particulier le taux de conversion par passe en lissant le profil thermique.

Ladite étape de déshydratation est alimentée par une alimentation de déshydratation formée à partir d'au moins une charge n-butanol diluée avec au moins une partie de l'effluent eau purifiée issu de l'étape c).

De manière préférée, ladite alimentation de déshydratation est vaporisée totalement, puis surchauffé, par échange de chaleur indirect à contre-courant dans un évaporateur suivi d'un surchauffeur avec l'effluent de l'étape b) de déshydrogénation. La partie de l'effluent eau purifiée issu de l'étape c) présente dans ladite alimentation de déshydratation est choisie de telle sorte que l'écart de température dans l'évaporateur entre ladite alimentation de déshydratation et l'effluent de l'étape b) est compris entre 5 et 20°C, préférentiellement entre 10 et 15°C.

On appelle approche thermique cet écart de température dans l'évaporateur.

L'ajustement de ladite partie de l'effluent eau purifiée issu de l'étape c) mélangé à ladite charge n-butanol est un critère essentiel de la présente invention. Cette partie est choisie la plus importante possible, de manière à ce que l'approche thermique soit comprise entre 5 et 20°C, préférentiellement entre 10 et 15°C, de manière non seulement à maximiser l'échange de chaleur entre ladite alimentation de déshydratation et ledit effluent issu de l'étape b), mais également de manière à diluer le plus possible ladite charge n-butanol tout en assurant sa vaporisation totale dans l'évaporateur.

Ladite étape de déshydratation est opérée préférentiellement à une température comprise entre 300 et 450°C, de préférence comprise entre 325 et 425°C et de manière plus préférée comprise entre 350 et 400°C, à une pression totale comprise entre 0,2 et 2 MPa, de préférence comprise entre 0,2 et 1 MPa et de manière plus préférée comprise entre 0,2 et 0,7 MPa, et avec une vitesse pondérale horaire (pph), qui est définie comme étant le rapport du débit massique en n-butanol sur la masse de catalyseur, comprise entre 1 et 14 h⁻¹, de préférence comprise entre 2 et 8 h⁻¹.

Ladite étape de déshydratation mise en œuvre selon l'invention permet d'obtenir une conversion du n-butanol supérieure à 90%, de préférence supérieure à 95% et de manière plus préférée supérieure à 99%, avec une sélectivité vers les n-butènes (but-1-ène, cis-but-2-ène et trans-but-2-ène) supérieure à 95%, de préférence supérieures à 99%.

L'absence ou la très faible quantité d'iso-butène formé dans l'étape de déshydratation est très avantageuse, ledit iso-butène étant préjudiciable aux performances de la déshydrogénation.

Dans un arrangement particulier, ledit catalyseur de déshydratation comprend une alumine (A) ayant une surface spécifique BET comprise entre 200 et 350 m²/g, avantageusement comprise entre 200 et 280 m²/g, et de manière préférée entre 200 et 230 m²/g, un diamètre moyen mésoporeux compris entre 5 et 15 nm, avantageusement compris entre 6 et 12 nm, et de manière préférée compris entre 7 et 11 nm, une teneur en sodium inférieure à 50 ppm poids et une teneur en soufre inférieure à 40 ppm poids. Dans cet arrangement, ledit catalyseur est avantageusement constitué de ladite alumine (A).

Dans un autre arrangement particulier, ledit catalyseur de déshydratation comprend une alumine (B) ayant une surface spécifique BET comprise entre 130 et 180 m²/g, avantageusement comprise entre 150 et 180 m²/g, un diamètre moyen mésoporeux compris entre 14 et 20 nm, avantageusement compris entre 15 et 20 nm, une teneur en sodium comprise entre 300 et 600 ppm poids et une teneur en soufre comprise entre 800 et 1300 ppm poids. Dans cet arrangement, ledit catalyseur est avantageusement constitué de ladite alumine (B).

La surface spécifique BET est mesurée selon la norme ASTM D3663-03. La détermination du diamètre moyen mésoporeux est faite par porosimétrie au mercure selon la norme ASTM D 4284-03 avec un angle de contact de 140°. L'analyse de porosimétrie au mercure correspond à l'intrusion d'un volume de mercure caractéristique de l'existence de mésopores et de macropores dans ledit catalyseur selon la norme ASTM D4284-03, les pores étant supposés de forme cylindrique. Cette technique permet d'accéder à la valeur du volume mercure mésoporeux défini comme étant le volume de mercure adsorbé par l'ensemble des pores ayant un diamètre compris dans la gamme des mésopores, à savoir compris entre 2 et 50 nm. Ce diamètre moyen mésoporeux est obtenu à partir de la courbe dérivée dV/dlog(D) (V étant le volume de mercure adsorbé et D le diamètre des pores) en fonction du diamètre des pores D et correspond à l'ordonnée pour laquelle l'abscisse dV/dlog(D) est maximal.

Ledit catalyseur comprenant ladite alumine (A) ou ledit catalyseur comprenant ladite alumine (B) peut également comprendre au moins une matrice de type oxyde, également appelée liant. Ladite matrice comprend avantageusement au moins un élément choisi dans le groupe formé par les argiles (par exemple parmi les argiles naturelles telles que le kaolin ou la bentonite), la magnésie, les alumines, les silices, les silice-alumines, les aluminates, l'oxyde de titane, l'oxyde de bore, la zircone, les phosphates d'aluminium, les phosphates de titane, les phosphates de zirconium, et le charbon, pris seuls ou en mélange.

De préférence, ledit catalyseur de déshydratation est soumis à une étape de calcination avant sa mise en œuvre, ladite étape de calcination ayant pour objectif d'éliminer les espèces éventuellement adsorbées en surface. L'étape de calcination consiste, par exemple, à porter le catalyseur à une température d'au moins 500°C sous un flux d'air ou d'azote pendant au moins 1 heure.

Le catalyseur de déshydratation est avantageusement mis en forme sous la forme de grains de différentes formes et dimensions. Il est avantageusement utilisé sous la forme d'extrudés cylindriques ou polylobés tels que bilobés, trilobés, polylobés de forme droite ou torsadée, mais peut éventuellement être fabriqué et employé sous la forme de poudre concassée, de tablettes, d'anneaux, de billes, de roues, de sphères. De préférence, ledit catalyseur est sous forme d'extrudés.

De manière surprenante, le catalyseur de déshydratation mis en œuvre selon l'invention permet de capter les impuretés azotées et soufrées, tel que par exemple la 2-méthylpyridine, le 2-N-propylthiophène, le diméthyltrisulfure et le carbone disulfure, présentes dans la charge et pouvant nuire aux performances des catalyseurs de déshydrogénation et aux spécifications du 1,3-butadiène produit à l'issu de l'étape de déshydrogénation. De préférence, ledit catalyseur de déshydratation permet de capter plus de 80% des impuretés azotés et soufrés, de manière préférée plus de 90%, de manière encore plus préférée plus de 95%.

### Étape de déshydrogénation

Conformément à l'invention, ledit effluent butènes est envoyé, sans étape de séparation/purification, dans au moins un réacteur afin d'être converti en au moins un effluent butadiène.

Ladite étape de déshydrogénation peut être réalisée au sein d'un ou plusieurs réacteur(s) isotherme(s) ou adiabatique(s) comprenant un lit de catalyseur de déshydrogénation, ledit lit étant choisi parmi les lits fixe, mobile et fluidisé.

Ledit effluent butènes est mélangé avec un agent oxydant, par exemple l'oxygène gazeux et peut éventuellement être de nouveau chauffé, avant d'être alimenté dans ladite étape réactionnelle de déshydrogénation.

Ledit effluent butènes est avantageusement mélangé avec un diluant inerte, par exemple de la vapeur d'eau surchauffée ou de l'azote avant d'alimenter ladite étape réactionnelle de déshydrogénation réalisée selon le mode oxydant. Le ratio molaire diluant inerte/butènes est compris entre 1 et 50, de préférence entre 2 et 30, de manière plus préférée entre 4 et 15.

De préférence, ledit diluant inerte est de la vapeur d'eau surchauffée. L'utilisation de la vapeur d'eau surchauffée peut permettre de chauffer l'effluent butènes à la température d'entrée dans le réacteur de déshydrogénation et son utilisation comme diluant permet d'absorber une partie de la chaleur dégagée par la réaction et de se placer dans une zone d'opération de la réaction non explosive.

Tous les catalyseurs permettant la conversion des paraffines en oléfines et/ou des oléfines en dioléfines peuvent être utilisés dans le cadre de cette invention. Le réacteur de déshydrogénation comprend avantageusement un lit de catalyseur de déshydratation permettant de déshydrater les dernières traces de butanol éventuellement présentes dans ledit effluent butènes.

Ladite étape réactionnelle de déshydrogénation des n-butènes est réalisée selon la voie oxydante, se distinguant notamment par la présence d'un agent oxydant en contact avec les n-butènes.

Ladite étape réactionnelle de déshydrogénation est une étape de déshydrogénation oxydante consistant à mettre en contact ledit effluent butènes avec un agent oxydant, par exemple l'oxygène gazeux, et un catalyseur de déshydrogénation oxydante, dans laquelle, à l'issue de la réaction, sont produits du butadiène, de l'eau, éventuellement de l'hydrogène, éventuellement du monoxyde et du dioxyde de carbone, éventuellement des hydrocarbures contenant de 1 à 6 atomes de carbone, éventuellement des hydrocarbures contenant plus de 6 atomes de carbones, éventuellement des composés oxygénés, éventuellement du coke sur le catalyseur. La réaction de déshydrogénation oxydante est une réaction exothermique, et non équilibrée qui correspond schématiquement à la réaction suivante : C₄H₈ + ½ O₂ → C₄H₆ + H₂O. De ce fait, elle est réalisée à plus faible température que la déshydrogénation non oxydante, par exemple à une température supérieure à 300°C et inférieure à 700°C.

De manière avantageuse, ladite étape réactionnelle de déshydrogénation réalisée en mode oxydante est opérée à une température comprise entre 300 et 700°C, de manière préférée comprise entre 320 et 650°C, de manière encore plus préférée entre 350 et 600°C, à une pression totale comprise entre 0,01 et 1 MPa, de préférence comprise entre 0,04 et 0,4 MPa, de manière préférée entre 0,05 et 0,25 MPa, avec une vitesse volumique horaire (V.V.H), qui est définie comme étant le rapport du débit volumique horaire de n-butènes en m³/h à 25°C, 1 atm divisé par le volume de catalyseur en m³, comprise entre 1 et 1500 h⁻¹, de préférence comprise entre 100 et 800 h⁻¹, en présence d'un agent oxydant. Ce dernier peut être un gaz, par exemple O₂, air, ou un solide de type oxyde métallique, par exemple de l'oxyde de fer. Le ratio molaire O₂/butènes est généralement compris entre 0,1 et 3, de préférence entre 0,5 et 1. Dans le cas d'un agent oxydant de type oxyde métallique, la quantité d'agent oxydant à apporter dépend de la quantité d'oxygène atomique de l'oxyde métallique réellement disponible pour la réaction de déshydrogénation. Il est généralement considéré qu'entre 10 et 100 % de l'oxygène de l'agent oxydant sous forme d'oxyde métallique est disponible et que le ratio molaire oxygène disponible / butènes est compris entre 0,2 et 6, de préférence 1 et 2.

Le catalyseur de déshydrogénation oxydante peut être choisis parmi les oxydes simples, par exemple oxyde de fer, oxyde de vanadium, les oxydes mixtes, par exemple des oxydes de molybdate de bismuth, oxyde à base de vanadium, de pyrophosphates métalliques et de ferrite, les métaux nobles généralement associés à des supports oxydes.

Les catalyseurs à base de molybdate de bismuth, additivés d'éléments multiples, tels que Co, Fe, Ni, Sn, K, P, Zr, Cr, Si, Li, Pb, Cd, Sb, ont largement été étudiés et sont considérés comme des catalyseurs efficaces pour la déshydrogénation oxydante des n-butènes en 1,3-butadiène. Leur formulation est généralement composée de quatre éléments comprenant un métal divalent (MII), en particulier Ni ou Co, un métal trivalent (MIII), en particulier Fe, Bi et Mo. Un rapport MIII:Bi:Mo de 3:1:12 est généralement appliqué pour le système de Bi-Mo multi-éléments. Les catalyseurs à base de Bi-Mo multi-éléments sont abondamment décrits dans l'art antérieur. En particulier, le brevet US 3 764 632 montre que le 1,3-butadiène est obtenu avec un rendement par passe de 96%, et une sélectivité de 97% en utilisant un catalyseur comportant les éléments Ni, Co, Fe, Bi, P, K et Mo à 320°C, un temps de contact 2,5 secondes, avec un mélange de 1-butène : air : vapeur d'eau (proportion molaire 1:10:5).

Les catalyseurs à base de ferrites comptent également parmi les plus utilisés et étudiés des catalyseurs employés pour la production du 1,3-butadiène par la voie de déshydrogénation oxydante grâce à leurs activités élevées. La formule générale est de type MIIMₓIIIFe₂₋ₓO₄ qui inclus un métal divalent (MII), par exemple Zn, Mg, Mn, Ni, Co, Cu, un métal trivalent (MIII), par exemple Al, Cr, Mn, Co, Fe, et du Fer.

Selon un mode de réalisation préférée, les étapes de déshydratation et de déshydrogénation sont réalisées dans un ou plusieurs réacteurs successifs avec des échanges thermiques intermédiaires réalisés dans des équipements différents permettant de refroidir ledit effluent butadiène et de chauffer au moins un flux du procédé selon l'invention choisi parmi la charge n-butanol, l'alimentation du réacteur de l'étape de déshydratation et l'alimentation du réacteur de l'étape de déshydrogénation.

Selon un autre mode de réalisation, l'enchainement des étapes de déshydratation et de déshydrogénation est réalisé dans un équipement intégrant les deux étapes, tant au point de vue énergétique que des flux de matières. Par exemple, les réactions de déshydratation et de déshydrogénation sont réalisées dans un même réacteur contenant un ou plusieurs lits, par exemple fixe, de catalyseur de déshydratation suivi d'un ou plusieurs lits, par exemple fixe, de catalyseur de déshydrogénation. Les lits de catalyseurs sont idéalement disposés, par exemple avec des zones intermédiaires exemptes de catalyseur, pour permettre le chauffage ou le refroidissement de l'effluent gazeux sortant d'un lit catalytique. De préférence, l'effluent gazeux issu du dernier lit de catalyseur de déshydratation est mélangé avec un agent oxydant, par exemple l'air, éventuellement avec un flux gazeux de n-butènes non convertis, éventuellement avec de la vapeur surchauffée et est éventuellement chauffé à la température de la réaction de déshydrogénation. Par exemple, une configuration avantageuse peut être d'utiliser un réacteur contenant plusieurs lits fixes de catalyseurs de déshydratation et de déshydrogénation avec un système d'échange thermique interne, tel qu'un serpentin par exemple, idéalement situé entre chaque lit de catalyseur dans lequel circule un fluide caloporteur, par exemple un système de type pompe à chaleur. Le fluide caloporteur extrait les calories produites par la réaction exothermique de déshydrogénation oxydante tout en refroidissant l'effluent gazeux entre deux lits de catalyseur de déshydrogénation via un échange de chaleur gaz/liquide. Le fluide caloporteur chaud, éventuellement vaporisé et comprimé, est ensuite utilisé pour réchauffer l'effluent gazeux entre deux lits de catalyseurs de déshydratation. Le fluide caloporteur est alors refroidi, éventuellement détendu, et peut de nouveau être utilisé pour refroidir les effluents gazeux issus des réactions de déshydrogénation.

### Étape de séparation

Conformément à l'invention, l'effluent issu de l'étape b) est séparé en au moins 2 fractions dans une étape de séparation.

Ledit effluent issu de l'étape b) est refroidi par un échange thermique avec au moins un flux du procédé selon l'invention choisi parmi la charge n-butanol, l'alimentation du réacteur de l'étape de déshydratation et l'alimentation du réacteur de l'étape de déshydrogénation. Une partie de la vapeur d'eau contenue dans l'effluent issu de l'étape b) peut avantageusement être condensée au cours de ce refroidissement.

L'effluent issu de l'étape b), après refroidissement, est introduit dans une colonne de séparation gaz/liquide dans laquelle l'eau de dilution et l'eau générée par les réactions de déshydratation et déshydrogénation est condensée ce qui permet de séparer ledit effluent issu de l'étape b) en un effluent butadiène gazeux comprenant le 1,3-butadiène et un effluent liquide comprenant de l'eau avec quelques composés dissous tel que par exemple CO₂, des hydrocarbures, des composés oxygénés. Ledit effluent butadiène peut également comprendre des butènes non convertis, des sous-produits de la réaction de déshydrogénation tel que H₂, CO, CO₂, des hydrocarbures comprenant de 1 à 6 atomes de carbone, de l'azote et de l'oxygène, des composés acétyléniques, des composés chimiques oxygénés et de l'eau non condensée.

De préférence, l'effluent butadiène sort en tête de ladite colonne de refroidissement à une température comprise entre 20 et 80°C.

Une partie de l'effluent liquide est recyclée, après refroidissement, dans la colonne de séparation gaz/liquide. L'autre partie est envoyée dans une étape de purification permettant de récupérer un effluent eau purifiée pour être recyclée dans le procédé. Avantageusement, l'étape de purification est une colonne de distillation ou une colonne de strippage permettant de séparer en tête les composés plus légers que l'eau, en fond les composés plus lourds que l'eau et/ou les sels issus de la charge butanol et/ou de la neutralisation des boucles de recycle d'eau dans la colonne de séparation gaz/liquide, et en soutirage latéral un effluent eau purifiée liquide ou vapeur exempt d'impuretés pouvant être recyclé dans le procédé et/ou être utilisé comme source de vapeur surchauffée. Avantageusement, le rebouillage de la colonne de distillation ou de strippage est réalisé grâce à un échange de chaleur avec une partie de l'effluent liquide recyclée dans la colonne de séparation gaz/liquide.

Ledit effluent butadiène est ensuite envoyé vers une section de compression comprenant plusieurs étages de compression avec refroidissement inter-étage dans laquelle il est comprimé jusqu'à une pression finale comprise entre 0,7 et 3,5 MPa, de manière préférée entre 1,0 et 2,0 MPa. Cette opération permet de récupérer au moins un effluent liquide aqueux non miscible avec les hydrocarbures, et au moins un effluent gazeux comprenant les gaz tels que H₂, CO, CO₂, N₂, O₂, des hydrocarbures « légers » comprenant de 1 à 3 atomes de carbone ainsi que du 1,3-butadiène et éventuellement des butènes non condensés, et éventuellement un effluent liquide hydrocarbures en C4 comprenant majoritairement du 1,3-butadiène et éventuellement des butènes. Par majoritairement, on entend au moins 50% poids ou 50 % volume dudit effluent.

La température en sortie d'un étage de compression est inférieure à 150°C, de préférence inférieure à 120°C. Le refroidissement inter-étage est réalisé de manière à abaisser la température du flux alimentant l'étage suivant à une température comprise entre 20 et 80°C, de préférence entre 30 et 50°C. Il est suivi d'une séparation des effluents aqueux, éventuellement liquide hydrocarbure et gazeux dans un ballon séparateur (K.O. drum selon la terminologie anglo-saxonne) afin que seul un flux gazeux alimente l'étage suivant de compression.

L'effluent gazeux comprimé issu du dernier étage de compression est envoyé dans une section de séparation permettant d'extraire le 1,3-butadiène et éventuellement les butènes non convertis contenus dans ledit effluent gazeux comprimé. Cette étape de séparation peut être réalisée par différents procédés connus de l'Homme du métier, comme la séparation par membrane ou l'extraction par un solvant, et de préférence réalisée par une extraction au solvant.

Dans les cas d'une extraction au solvant, ledit solvant est choisi parmi les hydrocarbures de type paraffine, oléfine, aromatique seuls ou en mélange, comprenant au moins 4 atomes de carbone. De manière avantageuse, ladite charge n-butanol est utilisée comme solvant d'extraction.

Ladite extraction au solvant est de préférence conduite à une température comprise entre 30 et 120°C et une pression comprise entre 0,7 et 3,5 MPa, de manière préférée entre 1,0 et 2,0 MPa, et de préférence selon un mode d'extraction à contre-courant.

Le taux de solvant, défini comme le ratio du débit volumique de solvant sur le débit volumique d'effluent gazeux entrant dans l'étape d'extraction, dépend des conditions de pression et de température, et du taux de récupération du 1,3-butadiène et éventuellement des butènes non convertis dans le solvant. Ce dernier est défini comme le ratio du débit molaire de butènes ou du 1,3-butadiène dans la phase solvant en sortie d'étape d'extraction sur le débit de butènes ou 1,3-butadiène dans l'effluent gazeux en entrée de l'extraction. Le taux de récupération des butènes et du 1,3-butadiène est idéalement supérieur à 90 %, de préférence supérieure à 95%, de manière plus préférée supérieure à 99%.

Pour une étape de déshydrogénation réalisée selon le mode oxydant, les composés oxygénés présent dans l'effluent en tête de la colonne de séparation sont extraits dans une étape intermédiaire avant l'étape d'extraction, par exemple par une étape de lavage à l'eau. Cette étape est réalisée entre 20 et 80°C et entre 0,4 et 2 MPa, de préférence entre 30 et 70°C et entre 0,6 et 1 MPa.

De l'étape d'extraction, on récupère un flux liquide contenant le 1,3-butadiène et éventuellement les butènes non convertis avec une faible quantité d'hydrocarbures comprenant entre 1 et 3 atomes de carbone et des gaz dissout, éventuellement des composés hydrocarbonés ayant plus de 4 atomes de carbones et le solvant d'extraction. Cet effluent est avantageusement mélangé à l'effluent liquide d'hydrocarbures en C4 éventuellement récupéré après refroidissement dans la section de compression.

Le flux liquide contenant le 1,3-butadiène et éventuellement les butènes non convertis, éventuellement en mélange à l'effluent liquide d'hydrocarbures en C4 récupéré après refroidissement dans la section de compression, est ensuite envoyé dans une étape de séparation permettant de séparer un effluent gazeux contenant les composés comprenant moins de 3 atomes de carbone et les gaz incondensables et un effluent liquide contenant le 1,3-butadiène, éventuellement les butènes non convertis, éventuellement des hydrocarbures comprenant plus de 4 atomes de carbone et éventuellement le solvant d'extraction. Avantageusement cette première étape de séparation est réalisé dans une colonne de distillation de type dépropaniseur. Le flux gazeux sortant en tête de ladite colonne de distillation de type dépropaniseur peut éventuellement contenir encore une quantité de 1,3-butadiène et de butènes non convertis. Ce flux gazeux peut alors être recyclé dans la section de compression pour maximiser la récupération des butènes et du 1,3-butadiène dans l'étape d'extraction.

L'effluent sortant en fond de ladite colonne de distillation de type dépropaniseur est ensuite envoyé dans une seconde étape de séparation qui est avantageusement réalisée dans une colonne de distillation, de type dépentaniseur, permettant de récupérer en tête une coupe C4 contenant le 1,3-butadiène et éventuellement les butènes non converti, et en fond un effluent liquide contenant des hydrocarbures comprenant plus de 5 atomes de carbone et éventuellement le solvant d'extraction.

Dans le cas de configuration préférée d'une étape d'extraction par un solvant, l'effluent liquide sortant en fond de ladite colonne de distillation de type dépentaniseur et contenant le solvant d'extraction est refroidi et recyclé dans la colonne d'extraction. Eventuellement, tout ou une partie de l'effluent est purifié selon des procédés connus de l'homme du métier, par exemple via une ou plusieurs colonnes de distillation, avant d'être recyclé. Cette purification d'au moins une partie du solvant d'extraction permet de purger une partie des composés hydrocarbonés ayant plus de 5 atomes de carbone éventuellement des impuretés issus de l'étape de déshydrogénation et qui pourraient s'accumuler dans la boucle de séparation par extraction.

La coupe C4 est généralement envoyée dans une unité de purification connue de l'homme du métier, par exemple une distillation extractive, pour récupérer un flux de butadiène purifié répondant aux spécifications de pureté requises, par exemple pour une utilisation dans un procédé de polymérisation, et un flux de butènes non convertis pouvant être recyclé dans l'étape de déshydrogénation.

### Brève description des figures

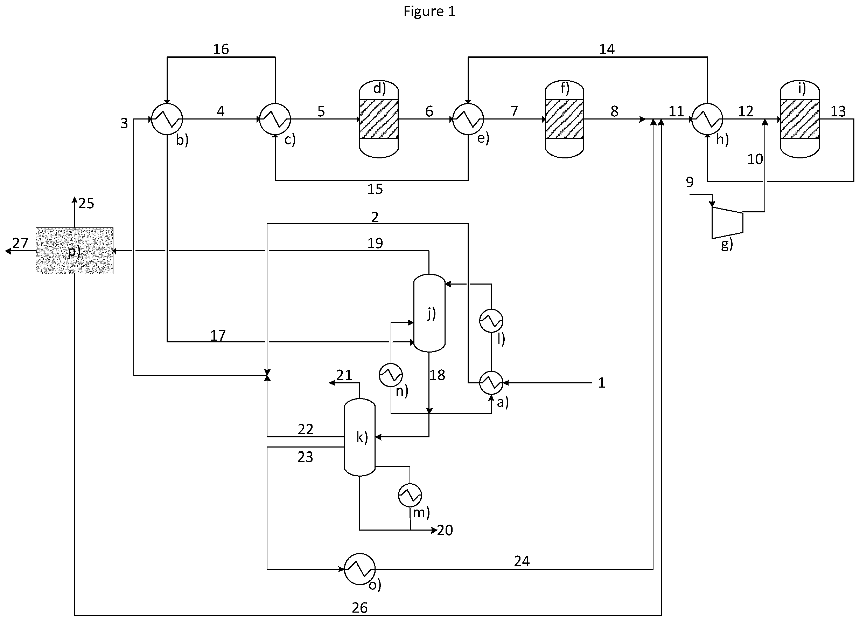

La figure 1 est un schéma représentant un mode particulier de réalisation de l'invention explicité à l'exemple 1.

### EXEMPLE

La charge n-butanol est produite par fermentation de glucose avec un mirco-organisme de type Clostridia produisant un mélange Acétone/Butanol/Ethanol selon un ratio de 3/6/1. Le n-butanol est ensuite récupéré via une série de distillation permettant d'atteindre une pureté supérieure à 99.8% en poids de n-butanol. La charge n-butanol est exempte d'iso-butanol et contient 0.005 % de composés azotés.

L'exemple de mise en œuvre du procédé selon l'invention est représenté à la figure 1.

### Section de déshydratation

Ladite charge n-butanol (1) est introduite à 35°C, à un débit de 32 614 kg/h, dans un échangeur a) à une pression de 0,47 MPa et est chauffé en restant en phase liquide jusqu'à une température de 96°C contre une partie de l'effluent aqueux (18) sortant en fond de la colonne de refroidissement j). La charge n-butanol chauffée (2) est ensuite mélangée avec 36 030 kg/h d'un flux d'eau liquide à 99°C et une pression de 0,42 MPa (22) soutiré de l'unité de purification k) (water stripper). Le mélange liquide n-butanol/eau (3) est introduit à 93°C dans l'échangeur de vaporisation b) de type rebouilleur et est totalement vaporisé grâce à un échange de chaleur avec l'effluent (16) issu du réacteur de déshydrogénation i) après passage dans les échangeurs c), e) et h).

Dans ledit échangeur de vaporisation de type rebouilleur, ledit mélange n-butanol/eau est vaporisé à une pression de 0,4 MPa pour donner un flux (4) à 137°C et l'effluent (16) à l'état gazeux, issu du réacteur de déshydrogénation, est refroidi sans être condensé pour donner un effluent (17) à une température de 147°C. Le débit d'eau liquide (22) soutiré de l'unité de purification k) a été ajusté de telle manière que l'approche thermique avec l'effluent issu du réacteur de déshydrogénation i) soit au minimum de 10°C avec une vaporisation totale du mélange n-butanol/eau.

Le mélange n-butanol/eau vaporisé est ensuite chauffé dans un échangeur c) de type monophasique gaz, grâce à un échange de chaleur avec l'effluent (15) issu du réacteur de déshydrogénation i) après passage dans les échangeurs e) et h), de manière à amener le mélange n-butanol/eau à une température d'entrée dans le premier réacteur de déshydratation compatible avec la température de la réaction de déshydratation. Dans ledit échangeur de type monophasique gaz, ledit mélange n-butanol/eau vaporisé est surchauffé à une température de 350°C et l'effluent (15) issu du réacteur de déshydrogénation est refroidi à 370°C pour donner l'effluent (16).

La première étape réactionnelle du procédé est une étape de déshydratation du n-butanol en n-butène. La réaction de déshydratation est endothermique. Dans l'exemple, l'étape de déshydratation comporte deux réacteurs de déshydratation adiabatiques d) et f) avec un échangeur e) intermédiaire de type monophasique gaz. Dans ledit échangeur de type monophasique gaz, l'effluent (6) sortant du premier réacteur de déshydratation d) à 303°C est chauffé à la température d'entrée dans le second réacteur de déshydratation f) de 350°C, grâce à un échange de chaleur avec l'effluent (14) issu du réacteur de déshydrogénation i) après passage dans l'échangeur h) qui est refroidi de 446 à 432°C.

Le mélange n-butanol/eau (5) vaporisé et chauffé à la température de la réaction de déshydratation, soit 350°C, est introduit dans le premier réacteur de déshydratation adiabatique d) à une pression de 0,39 MPa. La température et la pression de l'effluent (8) en sortie du second réacteur de déshydratation f) sont, respectivement, de 303°C et 0,32 MPa. La réaction de déshydratation est opérée, sur l'ensemble de deux réacteurs, à une vitesse pondérale horaire globale de 7 h⁻¹.

Les réacteurs adiabatiques de déshydratation contiennent un lit fixe de catalyseur de déshydratation C1, le dit catalyseur est une alumine γ cubique avec un diamètre moyen mésoporeux égal à 7,4 nm et une surface spécifique mesurée selon la norme ASTM D 3663-03 de 232 m²/g, contenant moins de 40 ppm de sodium et soufre.

La conversion totale du n-butanol à l'issu du second réacteur de déshydratation est de 99,85 %. La sélectivité en n-butènes, définie comme le ratio du nombre de mol de n-butènes produits par le nombre de mol de n-butanol converti, est de 99 %. Les coproduits de conversion du n-butanol sont de l'iso-butène et du butanal. La sélectivité des coproduits, définie comme le ratio du nombre de mol de coproduits par le nombre de mol de n-butanol converti, est de 0,8 % pour l'iso-butène et de 0,2 % pour le butanal. La répartition molaire des n-butènes est de 74,7 % de but-1-ène, 7,8 % de trans-but-2-ène et 17,5 % de cis-but-2-ène.

La quantité de composés azotés en sortie du second réacteur de déshydratation est nulle.

Pour comparaison, les performances d'un catalyseur de déshydratation C2, non conforme à une alumine (A) selon l'invention par sa surface spécifique et sa teneur en sodium et non conforme à une alumine (B) par son diamètre mésoporeux, sa surface spécifique et sa teneur en soufre, sont reportés dans le tableau 1.

**Tableau 1**

| Catalyseur | Type d'alumine | Diamètre moyen mésoporeux (nm) | Surface spécifique (m²/g) mesurée selon ASTM D 3663-03 | Teneur en sodium (ppm poids) | Teneur en soufre (ppm poids) |
|---|---|---|---|---|---|
| C1 (selon l'invention) | Alumine γ cubique | 7,4 | 232 | 35 | Inférieure à 40 |
| C2 (non conforme) | Alumine γ + Alumine δ | 8,4 | 193 | 500 | Inférieure à 40 |

### Section de déshydrogénation

L'effluent (8) issu du second réacteur de déshydratation f) est mélangé avec un flux à 112 451 kg/h d'eau de dilution vaporisée (24) à 350°C issue de la vaporisation de l'eau de recycle purifié (23) dans le four o) et avec un flux de n-butènes non convertis séparés de l'effluent réactionnel (19) dans l'étape de séparation p) et recyclés via le flux (26). Le débit d'eau de dilution (23) a été ajusté pour atteindre un ratio de dilution molaire n-butènes / eau, incluant l'eau initialement introduite via le flux (22) et l'eau formée par la réaction de déshydratation, de 12 dans le flux (12) en entrée du réacteur de déshydrogénation i). Le flux (11), issu du mélange de l'effluent (8) avec la vapeur de dilution (24) et le flux de n-butènes recyclés (26), est introduit à 308°C dans l'échangeur h) de type monophasique gaz et surchauffé à 380°C grâce à un échange de chaleur avec l'effluent réactionnel (13) issu du réacteur de déshydrogénation oxydante i). Le flux de n-butènes dilués et surchauffés (12) est mélangé en entrée du réacteur de déshydrogénation oxydante i) avec un flux d'air comprimé (10). La température du flux (12) a été ajusté pour atteindre une température en entrée de réacteur de déshydrogénation oxydante i) de 356 °C. L'air est apporté par le compresseur g) à une pression de 0,3 MPa. Le débit d'air du flux (9) a été ajusté pour atteindre un ratio molaire n-butènes / dioxygène en entrée de réacteur de déshydrogénation oxydante i) égal à 0,55.

Le mélange n-butènes/eau/oxygène est introduit dans le réacteur de déshydrogénation oxydante i) à une pression de 0,26 MPa. La pression de l'effluent (13) en sortie du réacteur de déshydrogénation oxydante i) est de 0,22 MPa. La réaction de déshydrogénation est opérée à une vitesse pondérale horaire de 1,14 h⁻¹.

Le réacteur de déshydrogénation oxydante i) contient un lit fixe de catalyseur de déshydrogénation oxydante, ledit catalyseur est une ferrite de type ZnFe₂O₄.

La conversion molaire des n-butènes dans le réacteur de déshydrogénation oxydante i) pour le cas 1 mettant en jeu le catalyseur C1 selon l'invention est de 59,4 % et la sélectivité en 1,3-butadiène, définie comme le ratio du nombre de mol de 1,3-butadiène produite par le nombre de mol de n-butènes convertis, est égale à 94,3 %.

Les coproduits sont du CO₂, H₂, CH₄, hydrocarbures en C2, C3, des acétylènes et des composés oxygénés issus de réaction de combustion, de craquage, de déshydrogénation poussée et d'oxydation partielle.

Pour comparaison, les performances de la déshydrogénation oxydante sont reportées dans le tableau 2 pour le cas 2 non conforme à l'invention mettant en jeu le catalyseur C2 et le cas 3 conforme à l'invention mettant en jeu le catalyseur C1 avec une alimentation supplémentaire (coprocessing selon la terminologie anglaise) d'une charge n-butènes non pure.

**Tableau 2: Performances du procédé**

| | | CAS 1 (selon l'invention) | | CAS 2 (comparatif) | | CAS 3 (selon l'invention) |
|---|---|---|---|---|---|---|
| | | A t₀ | Après 1 semaine | A t₀ | Après 1 semaine | A t₀ |
| Section déshydratation | | | | | | |
| Catalyseur | | C1 | C1 | C2 | C2 | C1 |
| Conversion n-butanol | (%) | 99,85 | 99,8 | 99,5 | 99,5 | 99,85 |
| Sélectivité n-butènes | (%) | 99 | 97,8 | 93,6 | 86,5 | 99 |
| Sélectivité iso-butène | (%) | 0,8 | 1,6 | 5,9 | 11,5 | 0,8 |
| Sélectivité en oxygénés (hors butanol) | (%) | 0,2 | 0,6 | 0,5 | 2,0 | 0,2 |
| | | | | | | |

| Section déshydrogénation | | | | | | |
|---|---|---|---|---|---|---|
| Catalyseur | | ZnFe₂O₄ | ZnFe₂O₄ | ZnFe₂O₄ | ZnFe₂O₄ | ZnFe₂O₄ |
| Charge en « coprocessing » n-butènes iso-butène | (kg /h) | - | - | - | - | 5000 |
| | (kg /h) | - | - | - | - | 250 |
| Conversion n-butènes | (%) | 59,4 | 58,7 | 56,0 | 53,0 | 58,9 |
| Sélectivité 1,3-butadiène | (%) | 94,3 | 94,1 | 89,8 | 86,3 | 94,2 |

Le catalyseur de déshydratation C2, non conforme à l'invention, entraine une formation d'iso-butène représentant 5,9 % en poids des butènes produits en sortie du dernier réacteur de déshydratation et 4,5 % en poids de la charge butènes (recycle inclut) entrant dans le réacteur de déshydrogénation oxydante. Cela se traduit sur les performances de la déshydrogénation oxydante par une baisse de la conversion de 3,4 % et une baisse de la sélectivité de 4,5 %.

On voit que les pertes de performances après une semaine de fonctionnement, la conversion en butanol étant maintenue dans l'étape de déshydration en élevant progressivement la température, sont bien plus forte dans le cas non-conforme. En effet, la capacité supérieure de captation des composés azotés dans le cas conforme à l'invention permet non seulement de maintenir une bonne sélectivité dans l'étape de déshydratation, mais également de protéger l'étape de déshydrogénation.

Le cas 3 montre que l'ajout supplémentaire d'une charge butènes contenant 5% en poids d'iso-butène impacte très faiblement les performances de la déshydrogénation oxydante. Par dilution avec la charge n-butènes issue du dernier réacteur de déshydratation ne contenant que 0,6% poids d'iso-butène, la teneur en iso-butène dans la charge butènes (recycle inclut) en entrée de réacteur de déshydrogénation oxydante est égale à 1,1 % en poids des butènes. La présence d'iso-butène dans la charge de déshydrogénation (généralement > 3% en poids des butènes) diminue ainsi les performances de la déshydrogénation.

La réaction de déshydrogénation oxydante, ainsi que certaines réactions secondaires telles que les réactions de combustion ou d'oxydation, sont exothermiques. L'effluent (13) sort du réacteur de déshydrogénation à une température de 505°C.

L'effluent réactionnel (13) subit ensuite les quatre échanges de chaleur précédemment décrits et est envoyé via le flux (17) dans la colonne de refroidissement j). Un effluent gazeux comprenant le 1,3-butadiène, les n-butènes non convertis, les composés légers, une partie des composés oxygénés, de l'eau vapeur, de l'oxygène non consommé et de l'azote, à une pression égale à 0,16 MPa et une température égale à 47°C est séparé en tête de colonne, ainsi qu'un effluent liquide comprenant de l'eau à 108°C en fond de colonne. Cette séparation est réalisée par l'utilisation d'une colonne de refroidissement, avec recyclage d'une partie de l'eau (18) récupérée en fond de colonne vers la tête et dans une zone intermédiaire de la colonne, après refroidissement et injection d'agent neutralisant.

Une partie de l'effluent (18) est refroidi jusqu'à 35°C dans les échangeurs a) et l) et recyclé en tête de colonne. Une seconde partie est refroidie dans l'échangeur n) à 50°C et recyclé sur un plateau intermédiaire de la colonne. Une troisième partie de l'effluent (18) est envoyée dans une colonne de purification k) dans laquelle les composés légers solubilisés dans l'eau sont éliminés en tête de colonne via le flux (21) et les composés lourds, les sels issus de la neutralisation de l'eau de refroidissement et l'eau en excédant formée au cours de la réaction de déshydrogénation sont purgés en fonds de colonne via le flux (20). De l'eau traitée et à son point de bulle est soutirée latéralement de la colonne de purification et renvoyée dans le procédé en mélange avec la charge n-butanol via le flux (22) et pour la génération de vapeur via le flux (23) et le four o).

L'effluent réactionnel refroidi en tête de la colonne de refroidissement (19) contenant le 1,3-butadiène, les n-butènes non converties, les produits de réactions secondaires tel que CO₂, H₂, CH₄, hydrocarbures en C2, C3, de l'eau, de l'azote, de l'oxygène ainsi que quelques composés oxygénés, est envoyé dans l'étape de séparation et de purification p).

Cette étape de séparation et purification comprend une section de compression de l'effluent réactionnel (19), une section de lavage à l'eau sous pression pour éliminer les composés oxygénés, une section d'absorption/régénération avec un solvant hydrocarboné permettant de séparer les composés légers jusqu'aux hydrocarbures en C3 et une coupe hydrocarbonée en C4 contenant le 1,3-butadiène, les n-butènes, les acétylènes et le hydrocarbures ayant plus de 4 atomes de carbone et une section de purification, comme par exemple une distillation / extraction avec un solvant de type NMP. L'étape de séparation et de purification p) permet de récupérer un flux de 1,3-butadiène (25) répondant aux spécifications de pureté requises (> 99,5%), un flux de n-butènes (26) recyclé dans le réacteur de déshydrogénation oxydante et une purge (27) contenant les composés légers, composés hydrocarbonés en C5+ et les composés oxygénés. Ce flux purge peut être utilisé comme combustible, par exemple, pour alimenter le four o) de génération de vapeur.

Les principaux flux, en kg/h, sont renseignés dans le tableau 3.

**Tableau 3 : Composition des principaux flux**

| Flux | | 1 | 3 | 4 | 5 | 8 | 9 | 12 | 13 | 17 |
|---|---|---|---|---|---|---|---|---|---|---|
| Température | °C | 35 | 93 | 137 | 350 | 303 | 35 | 380 | 505 | 147 |
| Eau | kg/h | 0 | 36031 | 36031 | 36031 | 43939 | 0 | 154783 | 161411 | 161411 |
| n-butanol | kg/h | 32614 | 32614 | 32614 | 32614 | 49 | 0 | 49 | 29 | 29 |
| n-butènes | kg/h | 0 | 0 | 0 | 0 | 24405 | 0 | 40129 | 16304 | 16304 |
| iso-butène | kg/h | 0 | 0 | 0 | 0 | 197 | 0 | 246 | 49 | 49 |
| 1,3-butadiène | kg/h | 0 | 0 | 0 | 0 | 0 | 0 | 442 | 22107 | 22107 |
| Autres (légers + oxygénés) | kg/h | 0 | 0 | 0 | 0 | 55 | 11035 | 55 | 6768 | 6768 |
| Total | kg/h | 32614 | 68645 | 68645 | 68645 | 68645 | 11035 | 195704 | 206669 | 206669 |
| | | | | | | | | | | |
| | | | | | | | | | | |
| Flux | | 18 | 19 | 20 | 21 | 22 | 24 | 25 | 26 | 27 |
| Température | °C | 108 | 47 | 95 | 99 | 99 | 350 | 42 | 42 | 40 |
| Eau | kg/h | 1573938 | 3448 | 36 | 11151 | 36030 | 110845 | 0 | 0 | 0 |
| n-butanol | kg/h | 17 | 27 | 0 | 2 | 0 | 0 | 0 | 0 | 0 |
| n-butènes | kg/h | 10 | 16303 | 1 | 0 | 0 | 0 | 108 | 15724 | 471 |
| iso-butène | kg/h | 0,1 | 49 | 0 | < 0,01 | 0 | 0 | 0 | 49 | 0 |
| 1,3-butadiène | kg/h | 16 | 22105 | 2 | 0 | 0 | 0 | 21663 | 442 | |
| Autres (légers + oxygénés) | kg/h | 12 | 6767 | 1 | 0 | 0 | 0 | 0 | 0 | 6794 |
| Total | kg/h | 1573993 | 48699 | 39 | 11153 | 36030 | 110845 | 21771 | 16215 | 7265 |

Cet exemple montre que la réalisation de la section de déshydratation avec un catalyseur de déshydratation sélectif et selon les conditions conformes à l'invention permet d'envoyer directement l'effluent réactionnel issu du dernier réacteur de déshydratation dans la section de déshydrogénation oxydante sans avoir à séparer et purifier préalablement les n-butènes produits dans la section de déshydratation. Cette intégration permet de supprimer le besoin de la colonne de refroidissement et de l'unité de purification (Water Stripper) des eaux usées pour l'étape de déshydratation soit un gain de 700 kW pour le rebouilleur du Water Stripper et de 27 MW pour le refroidissement des boucles d'eau de recycle sur la colonne de refroidissement.

De plus, les échangeurs b) c) e) h) permettent de récupérer 49 MW d'énergie thermique issue de la section de déshydrogénation oxydante pour réaliser la vaporisation et le chauffage de la charge n-butanol/eau de la section de déshydratation et le chauffage de la charge vapeur n-butènes/eau de la section de déshydrogénation oxydante. Par rapport à un procédé selon l'état de l'art en deux étapes indépendantes, le procédé intégré selon l'invention permet de gagner 29 MW d'énergie thermique, soit 25 % des besoins énergétiques du procédé selon l'état de l'art, en considérant l'étape de séparation et de purification p) identique dans l'étape de déshydrogénation oxydante et sans compter la part énergétique due à la séparation et purification des n-butènes dans l'étape de déshydratation.

De plus, le fait de ne pas condenser la vapeur de dilution de la section de déshydratation permet d'utiliser la vapeur d'eau produite par la réaction de déshydratation pour la dilution des n-butènes dans la section de déshydrogénation, soit une diminution de 7823 kg/h de vapeur externe à apporter ou générer par rapport à un procédé selon l'état de l'art en deux étapes indépendantes.

Le fait de mélanger la charge n-butanol avec de l'eau de recycle issue de l'unité de purification permet d'abaisser la température d'ébullition du n-butanol et ainsi d'atteindre une température de l'effluent gazeux en entrée de la colonne de refroidissement plus faible ce qui permet de réduire les débits d'eau de recycle dans la colonne de refroidissement. L'eau de dilution avec la charge n-butanol joue également le rôle de tampon thermique pour la réaction de déshydratation endothermique, permettant d'éviter des températures élevées (typiquement supérieure à 400°C) en entrée de réacteur de déshydratation et de fort gradient thermique préjudiciable à la sélectivité. Par ailleurs, l'ajustement du débit d'eau de dilution avec la charge n-butanol présente l'avantage de permettre une plus grande flexibilité dans la conduite de la section de déshydrogénation en contrôlant les variations d'énergie thermique générée (variation de la conversion et des sélectivités, variation des conditions opératoires, désactivation du catalyseur..) par la réaction de déshydrogénation oxydante.

De plus, le fait de produire une faible quantité d'iso-butène dans la section de déshydratation et la capacité du catalyseur de déshydratation à capter les impuretés azotés et soufrés présente l'avantage de pouvoir alimenter en supplément la section de déshydrogénation avec des charges butènes ne répondant pas aux spécifications de pureté requise pour la déshydrogénation oxydante, tel que des charges n-butènes contenant plus de 3 % en poids d'iso-butène.

## Revendications

1. Procédé de production de butadiène à partir de butanol comprenant au moins les étapes suivantes:
a) déshydratation dudit butanol, alimentée par une alimentation de déshydratation formée à partir d'au moins ladite charge n-butanol diluée avec au moins une partie de l'effluent eau purifiée issu de l'étape c), conduisant à un effluent butènes dans au moins un réacteur, en présence d'un catalyseur comprenant une alumine,
b) déshydrogénation oxydante dudit effluent butènes, dilué avec au moins une partie de l'effluent eau purifiée issu de l'étape c), en butadiène, ledit effluent butènes n'ayant subi aucun traitement à la suite de l'étape a) de déshydratation,
c) séparation de l'effluent issu de l'étape b) en au moins un effluent butadiène et un effluent eau purifiée,
dans lequel au moins une partie de l'énergie thermique nécessaire est apportée par la réaction de déshydrogénation de l'étape b), la température élevée de l'effluent butadiène en sortie de l'étape de déshydrogénation permettant de préchauffer ou chauffer les flux entrant et/ou sortant de l'étape a) de déshydratation et/ou de vaporiser la charge n-butanol et/ou de vaporiser une partie de l'eau de dilution de la charge.

2. Procédé selon la revendication 1, **caractérisé en ce que** ladite alimentation de déshydratation est vaporisée totalement, puis surchauffée, par échange de chaleur indirect avec l'effluent de l'étape b) de déshydrogénation, à contre-courant dans un évaporateur suivi d'un surchauffeur.

3. Procédé selon la revendication 2 dans lequel la partie de l'effluent eau purifiée issu de l'étape c) présente dans ladite alimentation de déshydratation est choisie de telle sorte que l'écart de température dans ledit évaporateur entre ladite alimentation de déshydratation et l'effluent de l'étape b) est compris entre 5 et 20°C.

4. Procédé selon l'une quelconque des revendications 1 à 3 à partir d'une charge comprenant au moins 60% poids de n-butanol choisi parmi le n-butan-1-ol, le n-butan-2-ol, et leurs mélanges.

5. Procédé selon l'une quelconque des revendications 1 à 4, dans lequel la première étape réactionnelle de déshydratation de la charge en un effluent butènes dans au moins un réacteur est opérée à une température à l'entrée de ladite étape réactionnelle de déshydratation comprise entre 300 et 450°C, à une pression comprise entre 0,2 et 2 MPa, à une vitesse pondérale horaire comprise entre 0,5 et 14 h⁻¹.

6. Procédé selon l'une quelconque des revendications 1 à 4 dans lequel la seconde étape réactionnelle de déshydrogénation oxydante de l'effluent butènes en butadiène, est opérée C avec un ratio molaire n-butènes/dioxygène compris entre 0,50 et 3 la température en entrée de ladite étape réactionnelle de déshydrogénation oxydante étant d'au moins 300°.

7. Procédé selon l'une quelconque des revendications précédentes, **caractérisé en ce que** le butanol provient de ressources renouvelables.

8. Procédé selon l'une quelconque des revendications précédentes **caractérisé en ce que** l'alumine comprise dans le catalyseur de déshydratation présente une surface spécifique BET comprise entre 200 et 350 m²/g, un diamètre moyen mésoporeux compris entre 5 et 15 nm, une teneur en sodium inférieure à 50ppm en poids et une teneur en soufre inférieure à 40 ppm en poids.

9. Procédé selon l'une quelconque des revendications précédentes, **caractérisé en ce que** l'effluent butènes est mélangé avec un diluant inerte selon un ratio molaire diluant inerte/butènes compris entre 1 et 50.

10. Procédé selon l'une quelconque des revendications précédentes, **caractérisé en ce que** la seconde étape de déshydrogénation est mise en œuvre avec un ratio molaire dioxygène/butènes compris entre 0,1 et 3.

## Patentansprüche

1. Verfahren zur Herstellung von Butadien aus Butanol, umfassend mindestens die folgenden Stufen:
a) Dehydrierung des Butanols, die durch eine Dehydrierversorgung versorgt wird, die mindestens aus der verdünnten n-Butanol-Charge mit mindestens einem Teil des Abflusses von gereinigtem Wasser aus Schritt c) versorgt wird, was zu einem Butenabfluss in mindestens einen Reaktor im Beisein eines Katalysators, umfassend Aluminiumoxid, führt,
b) oxydierende Dehydrierung des Butenabflusses, der mit mindestens einem Teil des Abflusses von gereinigtem Wasser aus Schritt c) verdünnt ist, zu Butadien, wobei der Butenabfluss keiner Behandlung nach dem Schritt a) der Dehydrierung unterzogen wurde,
c) Trennung des Abflusses aus Schritt b) in mindestens einen Butadienabfluss und einen Abfluss von gereinigtem Wasser,
bei dem mindestens ein Teil der notwendigen Wärmeenergie durch die Dehydrierungsreaktion aus Schritt b) geliefert wird, wobei es die hohe Temperatur des Butadienabflusses am Ausgang des Dehydrierungsschritts ermöglicht, die Eingangs- und/oder Ausgangsströme aus dem Dehydrierungsschritt a) vorzuerhitzen oder zu erhitzen und/oder die n-Butanol-Charge zu verdampfen und/oder einen Teil des Wassers zur Verdünnung der Charge zu verdampfen.

2. Verfahren nach Anspruch 1, **dadurch gekennzeichnet, dass** die Dehydrierversorgung zur Gänze verdampft, dann durch indirekten Wärmeaustausch mit dem Abfluss aus dem Dehydrierungsschritt b) im Gegenstrom in einem Verdampfer, gefolgt von einem Überhitzer, überhitzt wird.

3. Verfahren nach Anspruch 2, bei dem der Teil des Abflusses von gereinigtem Wasser aus Schritt c), der in der Dehydrierversorgung vorhanden ist, derart ausgewählt ist, dass die Temperaturabweichung im Verdampfer zwischen der Dehydrierversorgung und dem Abfluss aus Schritt b) zwischen 5 und 20 °C beträgt.

4. Verfahren nach einem der Ansprüche 1 bis 3, ausgehend von einer Charge, umfassend mindestens 60 Gew.-% n-Butanol, das unter n-Butan-1-ol, n-Butan-2-ol und ihren Gemischen ausgewählt ist.

5. Verfahren nach einem der Ansprüche 1 bis 4, bei dem der ersten Dehydrierreaktionsschritt der Charge in einen Butenabfluss in mindestens einen Reaktor bei einer Temperatur am Eingang des Dehydrierreaktionsschritts zwischen 300 und 450 °C, bei einem Druck zwischen 0,2 und 2 MPa, bei einer stündlichen Gewichts-Geschwindigkeit zwischen 0,5 und 14 h⁻¹ durchgeführt wird.

6. Verfahren nach einem der Ansprüche 1 bis 4, bei dem der zweite oxydierende Dehydrierreaktionsschritt des Butenabflusses in Butadien mit einem Molverhältnis n-Butene/Disauerstoff zwischen 0,50 und 3 durchgeführt wird, wobei die Temperatur am Eingang des oxydierenden Dehydrierreaktionsschritts mindestens 300 °C beträgt.

7. Verfahren nach einem der vorhergehenden Ansprüche, **dadurch gekennzeichnet, dass** das Butanol von erneuerbaren Ressourcen stammt.

8. Verfahren nach einem der vorhergehenden Ansprüche, **dadurch gekennzeichnet, dass** das Aluminiumoxid, das in dem Dehydrierkatalysator enthalten ist, eine spezifische Fläche BET zwischen 200 und 350 m²/g, einen durchschnittlichen mesoporösen Durchmesser zwischen 5 und 15 nm, einen Natriumgehalt unter 50 Gew.-ppm und einen Schwefelgehalt unter 40 Gew.-ppm aufweist.

9. Verfahren nach einem der vorhergehenden Ansprüche, **dadurch gekennzeichnet, dass** der Butenabfluss mit einem inerten Verdünner in einem Molverhältnis inerter Verdünner/Butene zwischen 1 und 50 gemischt wird.

10. Verfahren nach einem der vorhergehenden Ansprüche, **dadurch gekennzeichnet, dass** der zweite Dehydrierungsschritt mit einem Molverhältnis Disauerstoff/Butene zwischen 0,1 und 3 eingesetzt wird.

## Claims

1. Method for producing butadiene from butanol that comprises at least the following steps:
a) Dehydration of said butanol, fed by a dehydration feed that is formed from at least said n-butanol feedstock that is diluted with at least a portion of the purified water effluent that is obtained from step c), leading to a butene effluent in at least one reactor, in the presence of a catalyst that comprises an alumina,
b) Oxidizing dehydrogenation of said butene effluent, diluted with at least a portion of the purified water effluent that is obtained from step c), into butadiene, with said butene effluent not having undergone any treatment following the dehydration step a),
c) Separation of the effluent that is obtained from step b) into at least one butadiene effluent and one purified water effluent,
wherein at least a portion of the necessary thermal energy is supplied by the dehydrogenation reaction of step b), with the high temperature of the butadiene effluent exiting the dehydrogenation step making it possible to preheat or to heat the streams that enter and/or exit from the dehydration step a) and/or to vaporize the n-butanol feedstock and/or to vaporize a portion of the dilution water of the feedstock.

2. Method according to Claim 1, **characterized in that** said dehydration feed is vaporized totally, and then superheated, by indirect heat exchange with the effluent of the dehydrogenation step b), in counter-current in an evaporator that is followed by a superheater.

3. Method according to Claim 2, wherein the portion of the purified water effluent that is obtained from step c) that is present in said dehydration feed is selected in such a way that the temperature difference in said evaporator between said dehydration feed and the effluent of step b) is between 5 and 20°C.

4. Method according to any one of Claims 1 to 3 from a feedstock that comprises at least 60% by weight of n-butanol that is selected from among n-butan-1-ol, n-butan-2-ol, and mixtures thereof.

5. Method according to any one of Claims 1 to 4, wherein the first reaction step for dehydration of the feedstock into a butene effluent in at least one reactor is performed at an initial temperature of said dehydration reaction step of between 300 and 450°C, at a pressure of between 0.2 and 2 MPa, and at an hourly speed by weight of between 0.5 and 14 h⁻¹.

6. Method according to any one of Claims 1 to 4, wherein the second reaction step for oxidizing dehydrogenation of the butene effluent into butadiene is performed with an n-butene/dioxygen molar ratio of between 0.50 and 3, with the initial temperature of said reaction step of oxidizing dehydrogenation being at least 300°C.

7. Method according to any one of the preceding claims, **characterized in that** the butanol comes from renewable resources.

8. Method according to any one of the preceding claims, **characterized in that** the alumina in the dehydration catalyst has a BET specific surface area of between 200 and 350 m²/g, a mean mesopore diameter of between 5 and 15 nm, a sodium content of less than 50 ppm by weight, and a sulfur content of less than 40 ppm by weight.

9. Method according to any one of the preceding claims, **characterized in that** the butene effluent is mixed with an inert diluent according to an inert diluent/butene molar ratio of between 1 and 50.

10. Method according to any one of the preceding claims, **characterized in that** the second dehydrogenation step is implemented with a dioxygen/butene molar ratio of between 0.1 and 3.
